# EUROPÄISCHE PATENTSCHRIFT

(11) **EP 1 389 942 B1**
(45) Veröffentlichungstag und Bekanntmachung des Hinweises auf die Patenterteilung: **12.09.2012**
(21) Anmeldenummer: 02774013.3
(22) Anmeldetag: 29.04.2002
(51) Int. Cl.: A61B 1/247, A61B 1/04

(54) **DENTALE ODER ENDOSKOPISCHE KAMERA**
DENTAL OR ENDOSCOPIC CAMERA
CAMERA DENTAIRE OU ENDOSCOPIQUE

(30) Priorität: 26.05.2001 DE 10125772
(43) Veröffentlichungstag der Anmeldung: 25.02.2004
(73) Patentinhaber: Dürr Dental AG, 74321 Bietigheim-Bissingen (DE)
(72) Erfinder: THOMS, Michael, 74321 Bietigheim-Bissingen (DE)
(74) Vertreter: Ostertag, Reinhard
(86) Internationale Anmeldenummer: PCT/EP2002/004699
(87) Internationale Veröffentlichungsnummer: WO 2002/096277

(56) Entgegenhaltungen:
- WO-A-94/09694
- WO-A-99/06871
- US-A- 4 969 450
- US-A- 5 416 638
- US-A- 6 117 071

## Beschreibung

Die Erfindung betrifft eine dentale oder endoskopische Kamera gemäß dem Oberbegriff des Anspruches 1.

In der US-A-6 117 071 ist ein Endoskop beschrieben, bei welchem in einem Gehäuse eine objektseitige Linsenanordnung und eine wandlerseitige Linsenanordnung vorgesehen ist. Diese beiden Linsenanordnungen bilden eine zu untersuchende Gewebestelle auf einem Bildwandler ab. Die hintere Linsenanordnung umfasst zwei eng benachbarte Linsen, von denen zwei verkittet sind. Bei diesem bekannten Endoskop wird die Gesichtsfeldblende durch einen radial nach innen gekanteten vorderen Flansch der Halterung für die zweite Linsenanordnung gebildet.

In der US-A-5 416 638 ist ein Endoskop beschrieben, bei welchem ein Objektiv aus vier verschiedenen Linsen gebildet ist, die über eine modular aufgebaute Licht- übertragungsstrecke zu einem Okular führt. Die einzelnen Module der Bildübertragungsstrecke umfassen jeweils einen Lichtleiter und eine gekittete Doppellinse. Das Objektiv umfasst eingangsseitig eine plankonkave Linse, deren Achse zur Endoskopachse geneigt ist und ein Paar von Umlenkspiegeln, welche den Strahlengang auf die Endoskopachse umlenken. Eine zweite Linsengruppe des Objektives umfasst drei eng benachbarte Linsen, von denen die ersten beiden bikonvex sind, die letzte bikonkav ist.

Bei derartigen Endoskopen und anderen Kameras ist die Optik wie generell bei Kameras als telezentrische Optik aufgebaut. Um bei einer solchen Optik gute Abbildungseigenschaften zu gewährleisten, müssen die objektseitige Linsenanordnung, die mittlere Linsenanordnung und die wandlerseitige Linsenanordnung aus mehreren (in der Regel zwei) Einzellinsen aufgebaut werden. Aus diesem Grunde sind die Optiken für derartige Kameras teuer.

Durch die vorliegende Erfindung soll eine Kamera gemäß dem Oberbegriff des Anspruches 1 so weitergebildet werden, dass sie bei weiterhin guten Abbildungseigenschaften preiswerter hergestellt werden kann.

Diese Aufgabe ist erfindungsgemäß gelöst durch eine Kamera mit den im Anspruch 1 angegebenen Merkmalen.

Es wurde überraschend herausgefunden, dass man eine gute Gesamtabbildung bei einer solchen Kamera auch dann erhält, wenn man vom Prinzip des telezentrischen Strahlenganges abgeht, wie er sich an sich bei Kameras bewährt hat. Bei nicht telezentrischem Strahlengang, kann man bei dentalen und endoskopischen Kameras auch Linsenanordnungen verwenden, die jeweils für sich genommen keine besonders guten Abbildungseigenschaften aufweisen. Insbesondere kann man die einzelnen Linsenanordnungen auch durch einzelne Linsen realisieren. Hierdurch wird eine beträchtliche Kostenersparnis erhalten, da weniger Linsen benötigt werden und auch die Montage der Optik vereinfacht ist.

Legt man die Gesichtsfeldblende so, wie im Anspruch 1 angegeben, so hat man besonders gute Abbildungseigenschaften. Die dritte Linsenanordnung wird in ihrem mittleren Bereich genutzt, wo die Verzeichnungsfehler klein sind. Die mittlere Linsenanordnung wird auch in ihren Randbereichen genutzt. Sie braucht aber keine stark gekrümmten Oberflächen aufzuweisen, so dass auch die Nutzung der Randbereiche dieser Linsenanordnung nicht zu nicht akzeptablen Verzeichnungsfehlern führt.

Gemäß Anspruch 1 kann man die erste Linsenanordnung aus nur einer optischen Komponente aufbauen, die sehr einfache Geometrie aufweist.

Die Erfindung ist auch im Hinblick auf ein einfaches Reinigen und Sterilisieren der Kamera von Vorteil.

Vorteilhafte Weiterbildungen der Erfindung sind in Unter-ansprüchen angegeben.

Auch die Weiterbildungen der Erfindung gemäß den Ansprüchen 3 und 4 geben besonders einfache Möglichkeiten an, die mittlere Linsenanordnung bzw. die wandlerseitige Linsenanordnung zu realisieren.

Eine Kamera, wie sie in Anspruch 5 angegeben ist, eignet sich besonders gut zur Verwendung für dentale Zwecke, da die Betrachtungsrichtung zur Achse des Handstückes geneigt ist, insbesondere senkrecht auf dieser steht.

Eine Kamera gemäß Anspruch 6 kann sowohl zur Betrachtung eines Gegenstandes aus unmittelbarer Nähe als auch zur Betrachtung eines Gegenstandes aus größerer Entfernung verwendet werden. Bei einer dentalen Kamera kann der Arzt zum Beispiel Einzelheiten eines Zahnes oder einen Gesamtüberblick über das Gebiss aufnehmen.

Anspruch 7 gibt eine besonders einfache Möglichkeit der Einstellung des Aufnahmeabstandes.

Bei einer Kamera gemäß Anspruch 8 ist die Einstellung des Aufnahmeabstandes möglich, ohne dass ein bewegtes Teil durch die Wand des Kameragehäuses hindurchgeführt werden muss.

Bei einer Kamera gemäß den Ansprüch 9 und 10 ist eine gute Beleuchtung des vor der Kamera befindlichen Objektes gewährleistet.

Nachstehend wird die Erfindung anhand von Ausführungsbeispielen unter Bezugnahme auf die Zeichnung näher erläutert. In dieser zeigen:
- Figur 1:: eine axialen Schnitt durch eine dentale Kamera;
- Figur 2:: eine schematische Ansicht der Optik der dentalen Kamera nach Figur 1; und
- Figur 3:: eine ähnliche Ansicht wie Figur 1, in welcher jedoch eine abgewandelte Verstellmechanik für den Bildwandler gezeigt ist.

Die in der Zeichnung wiedergegebene dentale Kamera hat ein Gehäuse 10, das als Plastikspritzteil hergestellt ist. Das Gehäuse 10 ist als einstückiges Gehäuse wiedergegeben; es versteht sich, dass der Fachmann es je nach den Herstellungserfordernissen als mehrteiliges Gehäuse konstruieren kann, wobei die verschiedenen Gehäuseteile dann unter Zwischenschaltung von Dichtungen dicht miteinander verbunden werden oder miteinander verklebt oder verschweißt werden.

Das Gehäuse 10 hat einen Griffabschnitt 12, der im wesentlichen die Form einer an den Enden geschlossenen zylindrischen Hülse hat. Der Griffabschnitt 12 trägt an seinem freien Ende einen sich verjüngenden und abgewinkelten Gehäuseabschnitt 14, dessen nach unten gewandtes Ende durch ein Fenster 16 bündig und dicht verschlossen ist.

In dem Gehäuse 10 ist eine insgesamt mit 4 bezeichnete Optik angeordnet, die ein schematisch angedeutetet Objekt 6 (Zahn oder Kieferbogen) auf einen Bildwandler 8 abbildet. Bei dem Bildwandler 8 kann es sich um ein Farb-CCD handeln.

In dem abgewinkelten Teil des Gehäuseabschnittes 14 ist ein Umlenkspiegel 18 angeordnet, der unter 45 Grad zur Achse des Griffabschnittes 12 und zur Achse des Fensters 16 angestellt ist und auch als Umlenkprisma, beispielsweise als Rechtwinkel- oder Pentaprisma, ausgebildet sein kann.

Im Strahlengang hinter dem Umlenkspiegel 18 ist eine Linse 22 angeordnet, die eine konkave vordere Stirnfläche 24 und eine konvexe hintere Stirnfläche 26 aufweist.

Unter größerem Abstand von der Linse 22 ist eine Zwischenlinse 28 angeordnet, welche eine konvexe objektseitige Stirnfläche 30 und eine konvexe wandlerseitige Stirnfläche 32 aufweist.

Wiederum eine größere Strecke hinter der Zwischenlinse 24 ist eine wandlerseitige Linse 34 vorgesehen, die eine konvexe objektseitige Stirnfläche 36 und eine konvexe wandlerseitige Stirnfläche 38 aufweist.

Der Bildwandler 8 ist auf einem Schlitten 40 angeordnet, der durch auf der Innenseite des Gehäuses 10 vorgesehene Führungsrippen 42, 44 längs der Achse der Optik 4 bewegbar geführt ist. Auf der einen Längsfläche des Schlittens 40 ist eine Zahnstange 46 ausgebildet, die mit einem Zahnrad 48 kämmt, welches drehbar am Gehäuse 10 gelagert ist und mit einem Zahnradabschnitt durch das Gehäuse 10 nach außen übersteht. Durch Drehen des Zahnrades 48 kann somit der Bildwandler 8 längs der Achse der Optik 4 verstellt werden.

In dem Gehäuse 10 ist ein im wesentlichen in axialer Richtung verlaufender Kanal 50 vorgesehen, in welchem ein Lichtleiter 52 vorgesehen ist. Ein Endabschnitt des Kanales 50 und des Lichtleiters 52 sind so abgewinkelt, dass auf den Lichtleiter 52 gegebenes Licht den Lichtleiter 52 leicht geneigt zur Achse des Fensters 16 verlässt, wie bei 54 gezeigt.

Der Bildwandler 8 und der Lichtleiter 52 sind über eine in der Zeichnung nicht wiedergegebene (dort rechts zu denkende) Steckverbindung mit einem Versorgungsschlauch verbunden, der ein zu einer Bildauswerteelektronik führendes Kabel sowie einen weiteren Lichtleiter aufweist, der zu einer Kaltlichtquelle führt.

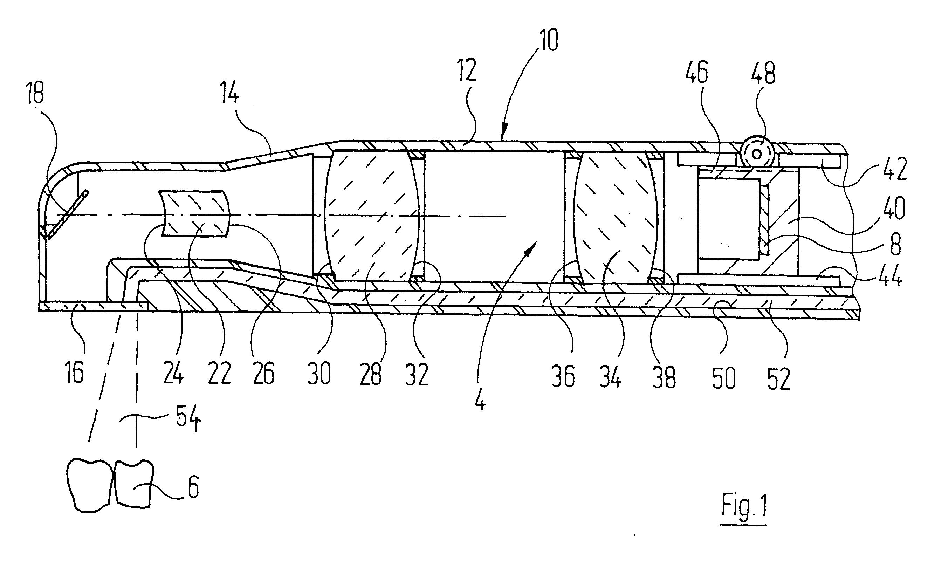

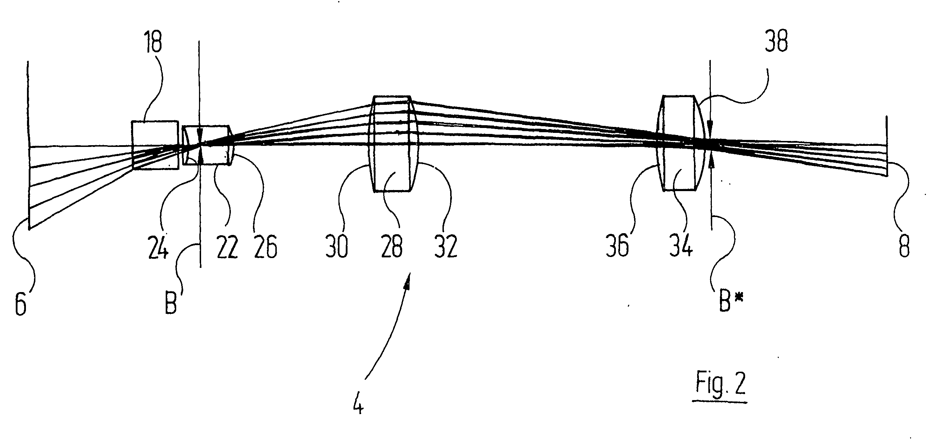

Der Strahlengang der Optik 4 ist in Figur 2 nochmals genauer gezeigt. Der besseren Darstellbarkeit halber wurden die Verhältnisse so gezeigt, wie sie bei einer Geradsicht-Kamera bestehen würde, die man aus der Kamera nach Figur 1 erhält, wenn man den als Umlenkprisma ausgebildeten Umlenkspiegel 18 durch eine planparallele Glasplatte gleicher optischer Dicke ersetzt und das Fenster 16 auf der Achse des Griffabschnittes 12 vorsieht. Die verschiedenen optischen Komponenten sind wieder so bezeichnet wie in Figur 1. Zusätzlich sind verschiedene Strahlen eingezeichnet, die von unterschiedlichen Punkten des Objektes 6 zu zugeordneten Punkten auf der Oberfläche des Bildwandlers 8 führen.

Man erkennt, dass bei der in Figur 2 gezeigten Optik der Gesichtsfeldblende B* ein Bild B konjugiert ist, welches in der Nähe der Linse 22 angeordnet ist. Man erkennt, dass bei solcher Lage der Gesichtsfeldblende B* bzw. des Bildes B derselben die objektseitige Linse 22 im wesentlichen in ihrem mittleren Bereich genutzt wird, die Zwischenlinse 28 auch in ihren Randbereichen genutzt wird und die wandlerseitige Linse 34 wieder nur in ihrem zentralen Bereich genutzt wird.

Aufgrund der gezeigten Anordnung der drei Linsen, bei welcher die Zwischenlinse 28 sowohl von der objektseitigen Linse 22 als auch von der wandlerseitigen Linse 34 deutlich beabstandet ist, braucht die Zwischenlinse 28 keine scharf gekrümmten Oberflächen zu haben. Hierdurch werden optische Aberrationen reduziert. Die Tatsache, dass in der Zwischenlinse 28 auch die Randbereiche genutzt werden, führt somit nicht zu einer nicht hinnehmbaren Verzeichnung des Bildes.

Die nachstehende Tabelle gibt ein konkretes Ausführungsbeispiel für eine Möglichkeit der Realisierung der Optik 4 an. Die Verhältnisse entsprechen der Darstellung von Figur 2.

Dabei ist jeweils aufgeführt die Nummer der Stirnfläche (Bezugszeichen von Figur 1 bzw. 2), der Krümmungsradius der entsprechenden Stirnfläche, die Dicke der Materialschicht, die sich an die Stirnfläche anschließt, und die Art des optischen Mediums (Glasart; L = Luft), welches hinter der entsprechenden Fläche liegt. In der letzten Spalte ist der Durchmesser der jeweiligen Stirnfläche angegeben. Längeneinheit ist jeweils 1 mm.

| Fläche | Radius | Dicke | Glas | Durchmesser |
|---|---|---|---|---|
| Objekt | ∞ | 9 | L | 13,21 |
| 19 | ∞ | 4 | SF8 | 3,80 |
| 21 | ∞ | 0,76 | L | 1,54 |
| 24 | -2,31 | 4,00 | N-LASF30 | 3,00 |
| 26 | -2,65 | 11,83 | L | 3,00 |
| 30 | 24,30 | 4 | N-LASF30 | 7,50 |
| 32 | -11,94 | 20,84 | L | 7,50 |
| 36 | 12,15 | 4,00 | N-ZK7 | 7,50 |
| 38 | -8,82 | 0,56 | L | 7,50 |
| Blende B^{*} | ∞ | 15,13 | L | 0,98 |
| Wandler | ∞ | | | 4,85 |

Soweit in der Spalte Glas "L" angegeben ist, handelt es sich um Luftstrecken. Die Glastypen entsprechen dem Katalog für optische Gläser der Firma Schott.

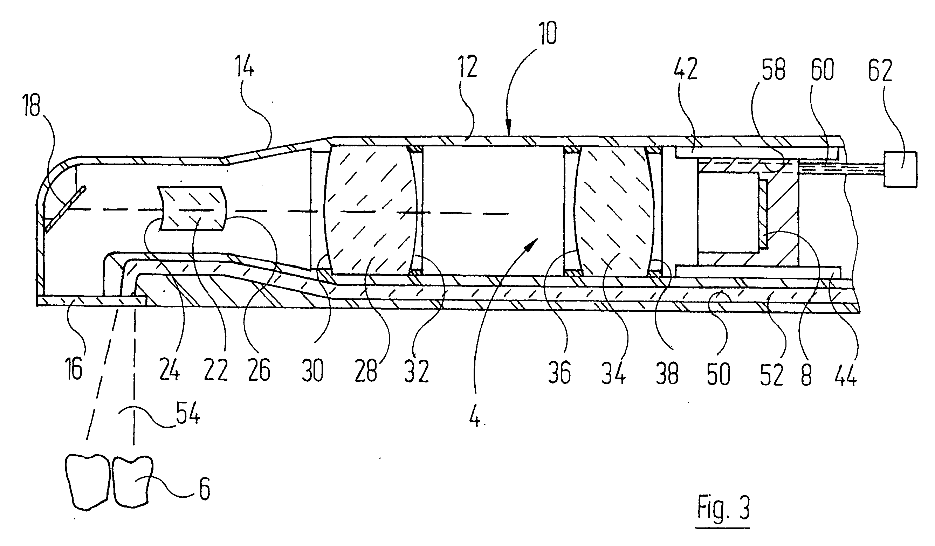

Das Ausführungsbeispiel nach Figur 3 entspricht weitgehend demjenigen nach Figur 1; entsprechende Komponenten sind wieder mit denselben Bezugszeichen versehen und werden nicht nochmals im einzelnen beschrieben.

Beim Ausführungsbeispiel nach Figur 3 ist der Schlitten 40 mit einer Gewindebohrung 58 versehen, in welcher eine Gewindelspindel 60 läuft. Die Gewindespindel 60 wird von einem Elektromotor 62 angetrieben, der vom Gehäuse 10 getragen ist. Die Versorgungsleitungen für den Elektromotor 62 gehen genauso über die rechts von Figur 3 zu denkende Steckverbindung zu einem Versorgungsschlauch wie die Anschlussleitungen des Bildwandlers 8 und der Lichtleiter 52.

Auf diese Weise kann der Wandler 8 längs der Achse der Optik 4 verstellt werden, ohne dass eine mechanische Durchführung durch die Wand des Gehäuses 10 vorgesehen werden müsste.

## Patentansprüche

1. Dentale oder endoskopische Kamera mit einem Gehäuse (10), mit einer Optik (4), mit einer das Bildfeld bestimmenden Blende und mit einem auf der Achse der Optik (4) angeordneten Bildwandler (8), wobei die Optik aus (4) einer objektseitigen Linsenanordnung (22), einer mittleren Linsenanordnung (28) und einer wandlerseitigen Linsenanordnung (34) besteht **dadurch gekennzeichnet, dass** die das Gesichtsfeld bestimmende Blende (B*) oder ein Bild (B) derselben im Bereich der wandlerseitigen Linsenanordnung (34) liegt, wobei sie um eine kleine Strecke hinter der wandlerseitigen Linsenanordnung (34) liegt, welche 2 bis 10% des Abstandes zwischen der hinteren Begrenzungsfläche (38) der wandlerseitigen Linsenanordnung (34) und der lichtempfindlichen Fläche des Bildwandlers (8) beträgt, dass ein vor der objektseiten Linsenanordnung (22) liegendes Eintrittsfenster (16) bündig und dicht mit dem Gehäuse (10) verbunden ist und dass die objektseitige Linsenanordnung (22) durch eine konkav/konvex gekrümmte Linse (22) gebildet ist.

2. Kamera nach Anspruch 1, **dadurch gekennzeichnet, dass**
die Blende (B*) um eine kleine Strecke hinter der wandlerseitigen Linsenanordnung (34) liegt, welche 2 bis 5% des Abstandes zwischen der hinteren Begrenzungsfläche (38) der wandlerseitigen Linsenanordnung (34) und der lichtempfindlichen Fläche des Bildwandlers (8) beträgt.

3. Kamera nach einem der Ansprüche 1 bis 2, **dadurch gekennzeichnet, dass** die mittlere Linsenanordnung (28) durch eine bikonvexe Linse gebildet ist.

4. Kamera nach einem der Ansprüche 1 bis 3, **dadurch gekennzeichnet, dass** die wandlerseitige Linsenanordnung (34) durch eine bikonvexe Linse gebildet ist.

5. Kamera nach einem der Ansprüche 1 bis 4, **gekennzeichnet durch** ein vor der objektseitigen Linsenanordnung (21) angeordnetes Lichtumlenkmittel (18).

6. Kamera nach einem der Ansprüche 1 bis 5, **gekennzeichnet durch** eine Einrichtung (46, 48; 60, 62) zum Verstellen des Bildwandlers (8) in Richtung der Achse der Optik (4).

7. Kamera nach Anspruch 6, **dadurch gekennzeichnet, dass** die Verstelleinrichtung ein durch eine Wand des Gehäuses (10) hindurchgeführtes Betätigungselement (48) aufweist.

8. Kamera nach Anspruch 6, **dadurch gekennzeichnet, dass** die Verstelleinrichtung einen Elektromotor (62) aufweist, der über eine Steckverbindung erregt wird, über welche auch der Bildwandler (8) mit einer Bildauswerteelektronik verbunden ist.

9. Kamera nach einem der Ansprüche 1 bis 8 **gekennzeichnet durch** einen im Gehäuse (10) verlaufenden Lichtleiter (52), dessen Lichtabgabeende einem Eintrittsfenster (16) des Gehäuses (10) benachbart ist.

10. Kamera nach einem der vorhergehenden Ansprüche, **gekennzeichnet durch** mehrere neben dem Eintrittsfenster (16) angeordnete Lichtquellen wie beispielsweise Leuchtdioden.

## Claims

1. A dental or endoscopic camera comprising a casing (10), with an optical system (4), with a stop determining the image field and with an image converter (8) arranged on the axis of the optical system (4), wherein the optical system (4) consists of an object-side lens arrangement (22), a middle lens arrangement (28) and a converter-side lens arrangement (34), **characterized in that** the stop (B*) determining the field of view or an image (B) of the same, is situated in the region of the converter-side lens arrangement (34), wherein it is situated a short distance downstream of the converter-side lens arrangement (34) amounting to 2% to 10%, of the spacing between the rear boundary face (38) of the converter-side lens arrangement (34) and the light-sensitive face of the image converter (8), **in that** an entrance window (16) situated upstream of the object-side lens arrangement (22) is connected to the casing (10) in a flush and tight manner and **in that** the object-side lens arrangement (22) is formed by a concavely/convexly curved lens (22).

2. Camera according to claim 1, **characterized in that** th stop (B*) is situated a short distance downstream of the converter-side lens arrangement (34) amounting to 2% to 5%, of the spacing between the rear boundary face (38) of the converter-side lens arrangement (34) and the light-sensitive face of the image converter (8).

3. Camera according to one of the claims 1 to 2, **characterized in that** the middle lens arrangement (28) is formed by a biconvex lens.

4. Camera according to one of the claims 1 to 3, **characterized in that** the converter-side lens arrangement (34) is formed by a biconvex lens.

5. Camera according to one of the claims 1 to 4, **characterized by** a light-reflecting means (18) arranged upstream of the object-side lens arrangement (21).

6. Camera according to one of the claims 1 to 5, **characterized by** a device (46, 48; 60, 62) for adjusting the image converter (8) in the direction of the axis of the optical system (4).

7. Camera according to Claim 6, **characterized in that** the adjusting device has an actuating element (48) which passes through a wall of the casing (10).

8. Camera according to Claim 6, **characterized in that** the adjusting device has an electric motor (62) which is excited via a plug-and-socket connection via which the image converter (8) is also connected to an image-evaluation circuit.

9. Camera according to one of the claims 1 to 8, **characterized by** an optical waveguide (52) extending within the casing (10), the light-output end of which is adjacent to an entrance window (16) of the casing (10).

10. Camera according to one of the preceding claims, **characterized by** several light-sources arranged next to the entrance window (16) as for example light-emitting diodes.

## Revendications

1. Caméra dentaire ou endoscopique comportant un boîtier (10), une optique (4), un diaphragme déterminant un champ d'image et un convertisseur d'image (8) disposé sur l'axe de l'optique (4), où l'optique (4) se compose d'un système de lentille (22) côté objet, d'un système de lentille (28) médian et d'un système de lentille côté convertisseur (34), **caractérisée en ce que** le diaphragme (B*) déterminant le champ visuel ou une image (B) de celui-ci se trouve dans la région du système de lentille côté convertisseur (34), où il repose derrière le système de lentille côté convertisseur (34) sur une petite trajectoire, qui correspond à 2 à 10 % de l'écart entre la surface de limitation arrière (38) du système de lentille côté convertisseur (34) et la surface photosensible du convertisseur d'image (8), **en ce qu'**une fenêtre d'entrée (16) placée devant le système de lentille côté objet (22) est reliée de façon étanche et affleurante au boîtier (10), et **en ce que** le système de lentille côté objet (22) est formé par une lentille à courbure concave/convexe (22).

2. Caméra selon la revendication 1, **caractérisée en ce que** le diaphragme (B*) repose derrière le système de lentille côté convertisseur (34) sur une petite trajectoire, qui correspond à 2 à 5 % de l'écart entre la surface de limitation arrière (38) du système de lentille côté convertisseur (34) et la surface photosensible du convertisseur d'image (8).

3. Caméra selon l'une des revendications 1 à 2, **caractérisée en ce que** le système de lentille médian (28) est formé par une lentille biconvexe.

4. Caméra selon l'une des revendications 1 à 3, **caractérisée en ce que** le système de lentille côté convertisseur (34) est formé par une lentille biconvexe.

5. Caméra selon l'une des revendications 1 à 4, **caractérisé par** un moyen de déviation de la lumière (18) disposé devant le système de lentille côté objet (21).

6. Caméra selon l'une des revendications 1 à 5, **caractérisé par** un dispositif (46, 48 ; 60, 62) de réglage du convertisseur d'image (8) dans la direction de l'axe de l'optique (4).

7. Caméra selon la revendication 6, **caractérisée en ce que** le dispositif de réglage présente un élément d'actionnement (48) guidé par une paroi du boîtier (10).

8. Caméra selon la revendication 6, **caractérisée en ce que** le dispositif de réglage présente un moteur électrique (62) excité par l'intermédiaire d'une connexion par fiche, par laquelle le convertisseur d'image (8) est aussi relié à une platine électronique de restitution des images.

9. Caméra selon l'une des revendications 1 à 8, **caractérisée par** une fibre optique (52) s'étendant dans le boîtier (10) et dont l'extrémité émettrice de lumière jouxte une fenêtre d'entrée (16) du boîtier (10).

10. Caméra selon l'une des revendications précédentes, **caractérisée par** plusieurs sources lumineuses disposées près de la fenêtre d'entrée (16), comme par exemple des diodes lumineuses.
